# EUROPEAN PATENT APPLICATION

(11) **EP 4 349 351 A1**
(43) Date of publication of application: **10.04.2024**
(21) Application number: 22816403.4
(22) Date of filing: 27.05.2022
(51) Int. Cl.: A61K 35/747, A61K 9/00, A61P 37/00, A61P 3/00, A23L 33/135, A61K 8/99, A61Q 19/00

(54) **LACTOBACILLUS PLANTARUM-DERIVED VESICLE AND USE THEREOF**

(30) Priority: 03.06.2021 KR 20210072078; 21.10.2021 KR 20210140918
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2022/007600
(87) International publication number: WO 2022/255744

(57) **Abstract**

The present invention relates to Lactobacillus plantarum-derived vesicles and a use thereof and, more specifically, to a composition containing Lactobacillus plantarum-derived vesicles as an active ingredient for prevention, alleviation, or treatment of an immune disease or a metabolic disorder. The present inventors found that when Lactobacillus plantarum-derive vesicles were administered into an immune disease or metabolic disorder model, pathogenic factor-induced immune dysfunction and metabolite-induced metabolic dysfunction can be effectively inhibited. Thus, the Lactobacillus plantarum-derived vesicles according to the present invention are expected to find advantageous applications in the development of medical products or health function foods for preventing or treating immune diseases or metabolic disorders or for alleviating symptoms thereof.

## Description

### [Technical Field]

The present invention relates to a composition for preventing, alleviating, or treating an immune disease and a metabolic disorder caused by immune dysfunction and metabolic dysfunction, which includes *Lactobacillus plantarum*-derived vesicles as an active ingredient.

This application claims priority to and the benefit of Korean Patent Application Nos. 10-2021-0072078 and 10-2021-0140918 filed in the Korean Intellectual Property Office on June 3, 2021 and October 21, 2021, respectively, and all the contents disclosed in the specification and drawings of the applications are incorporated in this application.

### [Background Art]

Entering the 21^{st} century, the disease pattern has changed such that the significance of acute infectious diseases previously recognized as infectious diseases decreases, whereas chronic diseases caused by immune and metabolic dysfunction occurring in major organs of the body have become the major diseases that reduce the quality of life and determine the human lifespan. Such a change in disease pattern is closely related to changes in dietary pattern. In the past, when nutrition was insufficient, an acute infectious disease was the main cause of death, but recently, chronic diseases related to metabolic dysfunction caused by overnutrition due to excessively abundant nutrients have become a major problem.

Due to the metabolic dysfunction caused by overnutrition, fat accumulates not only in adipose tissue, where fat is normally stored, but also in blood vessels, the heart, the liver, the kidneys, and muscles, and thus lipotoxicity is caused, resulting in a metabolic disease. That is, blood lipids and fatty acids are increased by a carbohydrate or lipid metabolism disorder, due to the increased fatty acids in cells, an inflammatory response is induced by the fatty acids, and the inflammatory response leads to cellular aging and cell death, resulting in dysfunction in the cardiovascular system and organs such as the liver, kidneys, muscles, and brain. Diseases caused by such etiological causes include cardiovascular diseases such as arteriosclerosis, metabolic syndrome, and heart failure, liver diseases such as non-alcoholic steatohepatitis and cirrhosis, renal diseases such as chronic nephropathy and renal failure, musculoskeletal diseases such as gout, sarcopenia, and osteoporosis, and degenerative brain diseases such as Alzheimer's disease and Parkinson's disease.

Immunity is a cellular defense mechanism against biological, chemical, physical, and mental stress and occurs through innate immunity and adaptive immunity. Recently, it has been revealed that immune function is closely related to the metabolic function of cells, and in relation to the pathogenesis of immune dysfunction caused by metabolic dysfunction, intracytoplasmic metabolites such as fatty acids, uric acid, etc. act as danger signals and are recognized by a nucleotide-binding oligomerization domain (NLRP), which is a pattern recognition receptor that is present in the cytoplasm. NLRPs are known to induce apoptosis by forming inflammasomes and at the same time, secrete inflammatory mediators to induce inflammatory cell infiltration.

Meanwhile, it is known that the number of microorganisms that coexist in the human body reaches 100 trillion, which is larger than that of human cells, and the number of genes of microorganisms is 100-fold larger than that of humans. A microbiota or microbiome refers to a microbial community including bacteria, archaea and eukarya present in a given habitat.

Bacteria that coexist in our bodies and bacteria that exist in the surrounding environment secrete nanometer-sized vesicles to exchange information such as genes, low molecular compounds, and proteins with other cells. The mucosa forms a physical defense membrane through which particles having a size of 200 nanometers (nm) or more cannot pass, so that bacteria coexisting in the mucosa cannot pass through the mucosa, but bacteria-derived vesicles have a size of 200 nanometers or less, and thus relatively freely pass through epithelial cells via the mucosa to be absorbed in our bodies. As described above, although bacteria-derived vesicles are secreted from bacteria, they differ from bacteria in terms of their constituents, absorption rate in the body, and risk of side effects, and therefore, the use of bacteria-derived vesicles is completely different from that of living cells or has a significant effect.

Locally secreted bacteria-derived vesicles are absorbed by epithelial cells of the mucous membrane to induce a local inflammatory response, and the vesicles passing through the epithelial cells are systemically absorbed and distributed into each organ and control metabolic function and immune function in the organ in which the vesicles are distributed. For example, vesicles derived from pathogenic gram-negative bacteria such as *Escherichia coli* are pathogenic nanoparticles that locally trigger colitis or food poisoning, and when absorbed into blood vessels, are absorbed into vascular endothelial cells to induce an inflammatory response and promote a systemic inflammatory response and blood coagulation, and are absorbed into muscle cells where insulin acts to cause metabolic diseases such as insulin resistance and diabetes. On the other hand, vesicles derived from beneficial bacteria can control diseases by regulating abnormalities in immune and metabolic functions caused by pathogenic vesicles.

Bacteria of the genus *Lactobacillus* are known as representative beneficial bacteria that secrete lactic acid. Among them, *Lactobacillus plantarum* is a gram-positive bacillus that grow well not only in an anaerobic environment but also in an aerobic environment and a symbiotic bacterium in the vagina, mouth, skin, intestines, etc. In addition, bacteria of the genus *Lactobacillus* are known as bacteria that are important in the fermentation process. Gram-positive bacteria-derived vesicles such as *Lactobacillus plantarum* contain bacterial cell wall components, peptidoglycan and lipoteichoic acid, in addition to bacteria-derived proteins, metabolites, and nucleic acids.

However, no cases in which vesicles secreted from *Lactobacillus plantarum* are applied to prevent or treat diseases caused by immune and metabolic dysfunction have been reported.

### [Disclosure]

### [Technical Problem]

As a result of intensively conducting research to solve the above-mentioned conventional problems, the present inventors confirmed that *Lactobacillus plantarum-*derived vesicles have a therapeutic effect on an immune or metabolic disease caused by immune and metabolic dysfunction, and completed the present invention.

Therefore, the present invention is directed to providing a pharmaceutical composition for preventing or treating an immune disease or metabolic disease, comprising *Lactobacillus plantarum*-derived vesicles as an active ingredient.

The present invention is also directed to providing a food composition for preventing or alleviating an immune disease or metabolic disease, comprising *Lactobacillus plantarum*-derived vesicles as an active ingredient.

The present invention is also directed to providing a pharmaceutical composition for preventing or treating aging, comprising *Lactobacillus plantarum-*derived vesicles as an active ingredient.

The present invention is also directed to providing a food composition for preventing or alleviating aging, comprising *Lactobacillus plantarum*-derived vesicles as an active ingredient.

The present invention is also directed to providing a cosmetic composition for preventing or alleviating skin aging, comprising *Lactobacillus plantarum*-derived vesicles as an active ingredient.

The present invention is also directed to providing a drug delivery composition for treating an immune disease or metabolic disease, comprising *Lactobacillus plantarum*-derived vesicles as an active ingredient.

However, a technical problem to be achieved by the present invention is not limited to the aforementioned problems, and the other problems that are not mentioned may be clearly understood by a person skilled in the art from the following description.

### [Technical Solution]

To achieve the purpose of the present invention, the present inventors confirmed effects through various experiments. More specifically, when *Lactobacillus plantarum* was cultured, and vesicles were isolated from the cell culture and orally administered to a mouse, the vesicles were absorbed through the gastrointestinal tract and distributed to organs such as the lungs, the liver, the kidneys, and the brain. In addition, when epithelial cells were treated with the vesicles, cell death caused by a biological pathogenic factor was inhibited in a dose-dependent manner. In addition, when the vesicles were orally administered to an *E. coli* mouse model induced by dextran, which is an abnormal metabolite produced by specific bacteria when sucrose was ingested, anatomical changes in the large intestine were significantly suppressed. In addition, when the vesicles were administered to nerve cells, the expression of a neurotrophin gene, which was suppressed by a stress hormone, significantly increased. In addition, the expression of a key anti-aging-related gene Sirt 1, which was suppressed by a stress hormone, significantly increased. In addition, it was confirmed that the vesicles enhance the activation of AMP-activated kinase (AMPK), which is key signaling that overcomes a metabolic stress environment where ATP production does not well occur.

Thus, the present invention is provides a pharmaceutical composition for preventing or treating an immune disease or metabolic disease, comprising *Lactobacillus plantarum*-derived vesicles as an active ingredient.

In one embodiment of the present invention, the immune disease or metabolic disease may be selected from one or more gastrointestinal diseases selected from the group consisting of gastritis, peptic ulcers, celiac disease, and inflammatory bowel disease;
one or more oral diseases selected from the group consisting of gingivitis, periodontitis, and caries;
one or more female vaginal diseases selected from the group consisting of candida vaginitis, atrophic vaginitis, bacterial vaginitis or vaginosis, and trichomonas vaginitis;
one or more liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more lung diseases selected from the group consisting of chronic obstructive pulmonary disease, chronic interstitial pneumonitis, emphysema, bronchopulmonary dysplasia, and idiopathic pulmonary fibrosis;
one or more cardiovascular diseases selected from the group consisting of hyperinsulinemia, dyslipidemia, arrhythmia, atherosclerosis, angina, metabolic syndrome, myocardial infarction, stroke, and cardiomyopathy;
one or more renal diseases selected from the group consisting of glomerulonephritis, and chronic nephropathy;
one or more musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis; and
one or more neurological diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy, but the present invention is not limited thereto.

As yet another exemplary embodiment of the present invention, the vesicles may have an average diameter of 10 to 1000 nm, but are not limited thereto.

In yet another embodiment of the present invention, the vesicles may be isolated from a culture medium of *Lactobacillus plantarum,* but are not limited thereto.

In yet another embodiment of the present invention, the vesicles may be isolated from food manufactured by adding *Lactobacillus plantarum,* but the present invention is not limited thereto.

As another exemplary embodiment of the present invention, the vesicles may be naturally secreted or artificially produced from *Lactobacillus plantarum,* but the present invention is not limited thereto.

In addition, the present invention is provides a food composition for preventing or alleviating an immune disease or metabolic disease, comprising *Lactobacillus plantarum*-derived vesicles as an active ingredient.

In addition, the present invention is provides a pharmaceutical composition for preventing or treating aging, comprising *Lactobacillus plantarum*-derived vesicles as an active ingredient.

In addition, the present invention is provides a food composition for preventing or alleviating aging, comprising *Lactobacillus plantarum*-derived vesicles as an active ingredient.

In addition, the present invention is provides a cosmetic composition for preventing or alleviating skin aging, comprising *Lactobacillus plantarum*-derived vesicles as an active ingredient.

In addition, the present invention is provides a composition for delivering a drug for treating an immune disease or metabolic disease, comprising *Lactobacillus plantarum*-derived vesicles as an active ingredient.

In one embodiment of the present invention, the food composition may be a health functional food composition, but is not limited thereto.

In addition, the present invention provides a method for preventing or treating an immune disease or metabolic disease, the method comprising administering a composition comprising vesicles derived from *Lactobacillus plantarum* as an active ingredient to a subject in need thereof.

In addition, the present invention provides a use of a composition comprising vesicles derived from *Lactobacillus plantarum* as an active ingredient for preventing or treating an immune disease or metabolic disease.

In addition, the present invention provides a use of vesicles derived from *Lactobacillus plantarum* for preparing a drug for treating an immune disease or metabolic disease.

In addition, the present invention provides a method for preventing or treating aging, the method comprising administering a composition comprising vesicles derived from *Lactobacillus plantarum* as an active ingredient to a subject in need thereof.

In addition, the present invention provides a use of a composition comprising vesicles derived from *Lactobacillus plantarum* as an active ingredient for preventing or treating aging.

In addition, the present invention provides a use of vesicles derived from *Lactobacillus plantarum* for preparing a drug for treating aging.

In addition, the present invention provides a method of delivering a drug for treating an immune disease or metabolic disease, which comprises administering a composition comprising *Lactobacillus plantarum*-derived vesicles carrying a desired immune disease or metabolic disease drug as an active ingredient to a subject in need thereof.

In addition, the present invention provides a use of a composition comprising *Lactobacillus plantarum*-derived vesicles as an active ingredient for delivering a drug for treating an immune disease or metabolic disease.

In addition, the present invention provides a use of *Lactobacillus plantarum-*derived vesicles for preparing a preparation for delivering a drug for treating an immune disease or metabolic disease.

### [Advantageous Effects]

As the present inventors confirmed that *Lactobacillus plantarum*-derived vesicles are absorbed through the gastrointestinal tract and distributed to organs such as the lungs, the liver, the kidneys, and the brain, inhibit cell death caused by a pathogenic factor in a dose-dependent manner, inhibit inflammatory mediator secretion mediated by a pathogenic factor in a dose-dependent manner, inhibit the occurrence of colitis in a colitis mouse model, activate AMP-activated kinase (AMPK) signaling that increases cellular homeostasis against metabolic stress, and significantly increase the expression of the SIRT gene, which is a key protein that inhibits neurotrophin expression and aging, the *Lactobacillus plantarum*-derived vesicles according to the present invention can be effectively used not only for developing medicines or health functional food for preventing or treating an immune or metabolic disease, or alleviating symptoms thereof but also effectively used as a drug delivery system for treating the disease.

### [Description of Drawings]

FIGS. 1A and 1B show (A) a set of whole body images and (B) a set of images showing the intensity of fluorescence in organs after extraction over time when administering *Lactobacillus plantarum* (*L. plantarum*)-derived vesicles to mice according to one embodiment of the present invention.
FIGS 2A and 2B show the degrees of death of skin epithelial cells by pathogenic bacteria *Staphylococcus aureus*-derived vesicles (Sa EV) (A) and *L. plantarum*-derived vesicles (Lp EV) (B), respectively, according to one embodiment of the present invention.
FIGS. 3A and 3B show the therapeutic effects of *L. plantarum*-derived vesicles (Lp EV) on the death of skin epithelial cells by pathogenic bacteria *Staphylococcus aureus*-derived vesicles (Sa EV) according to one embodiment of the present invention, wherein FIG. 3A shows the result before Sa EV treatment, and FIG. 3B shows the result of co-treatment with Sa EV and *L. plantarum*-derived vesicles (Lp EV).
FIG. 4 shows an experimental protocol for confirming a therapeutic effect after orally administering *L. plantarum*-derived vesicles in a colitis mouse model induced by orally administering an abnormal metabolite dextran (2.5% DSS) produced in the body by specific bacteria when sucrose is ingested.
FIG. 5A shows the anatomical change (length change) of the large intestine after directly administering *L. plantarum*-derived vesicles (*L. plantarum*) to colitis mouse models orally, and FIG. 5B is a representative image showing the change according to administration.
FIG. 6 shows the results of evaluating the expression of neurotrophins when co-administering *L. plantarum*-derived vesicles (L-EV) to nerve cell models where the expression of neurotrophins is suppressed by glucocorticoid hormone (GC), which is a stress hormone.
FIG. 7 shows the results of evaluating the expression of anti-aging related genes when co-administering *L. plantarum*-derived vesicles (L-EV) to nerve cell models where aging is accelerated by glucocorticoid hormone (GC), which is a stress hormone.
FIG. 8 shows the results of evaluating the degree of activation of AMPK signaling by treating muscle cells with insulin, metformin, or *L. plantarum*-derived vesicles (Lp EV).
FIGS. 9A and 9B show the inhibitory effects of *L. plantarum*-derived vesicles (Lp EV) on the secretion of inflammatory mediators (IL-6 and TNF-α) by *E. coli-*derived vesicles, which is a pathogenic factor, respectively.

### [Modes of the Invention]

The present invention relates to *L. plantarum*-derived vesicles and a use thereof.

Hereinafter, the present invention will be described in detail.

The present inventors confirmed that when *L. plantarum* was cultured, and vesicles were isolated from the cell culture and then administered orally to mice, the vesicles were absorbed through the gastrointestinal tract and distributed through blood vessels to organs such as the lungs, the liver, the kidneys and the brain (see Example 2).

In addition, it was confirmed that when epithelial cells were treated with the vesicles, cell death caused by *Staphylococcus aureus*-derived vesicles, which are biological pathogenic factors, was inhibited in a dose-dependent manner (see Example 3).

In addition, it was confirmed that when the vesicles were orally administered to a colitis mouse model induced by the ingestion of dextran, which is an abnormal metabolite of sucrose, an anatomical change by inflammation was significantly inhibited (see Example 4).

In addition, it was confirmed that when the vesicles were administered to nerve cells, the expression of a neurotrophin gene and a key anti-aging gene Sirt 1, which are suppressed by a stress hormone, was significantly increased (see Examples 5 and 6).

In addition, it was confirmed that when the vesicles were administered to muscle cells, similar to the anti-aging drug metformin, the phosphorylation of AMPK, which is key signaling, for inhibiting metabolic function, is increased (see Example 7).

In addition, it was confirmed that when the vesicles were administered to inflammatory cells, the secretion of inflammatory mediators (IL-6 and TNF-α) caused by pathogenic factors was inhibited in a dose-dependent manner (see Example 8).

Thus, the present invention is provides a pharmaceutical composition for preventing or treating an immune disease or metabolic disease, comprising *Lactobacillus plantarum*-derived vesicles as an active ingredient.

As used herein, the term "extracellular vesicle" or "vesicle" refers to a structure formed of a nano-sized membrane secreted from various bacteria, and includes, for example, a vesicle derived from gram-negative bacteria such as *E. coli,* which has, an endotoxin (lipopolysaccharide), a toxic protein, and both bacterial DNA and RNA, or a vesicle derived from gram-positive bacteria such as bacteria of the genus *Micrococcus,* which have outer membrane vesicles (OMVs), a protein and a nucleic acid as well as components of a bacterial cell wall, such as peptidoglycan and lipoteichoic acid.In the present invention, the vesicles encompasses all structures which are naturally secreted from *Lactobacillus plantarum,* or formed of an artificially produced membrane.

The vesicles may be isolated by heat treatment or autoclaving during *Lactobacillus plantarum* culture, or using one or more methods selected from the group consisting of centrifugation, ultracentrifugation, autoclaving, extrusion, sonication, cell lysis, homogenization, freezing-thawing, electroporation, mechanical degradation, chemical treatment, filtration with a filter, gel filtration chromatography, pre-flow electrophoresis, and capillary electrophoresis of the cell culture. In addition, for isolation, washing for removing impurities, and concentration of the obtained vesicles may be further performed.

A method of isolating vesicles from the *L. plantarum* culture or fermented food according to the present invention is not particularly limited as long as the culture or fermented food contains vesicles. For example, vesicles may be isolated using a method such as centrifugation, ultracentrifugation, filtration by a filter, gel filtration chromatography, free-flow electrophoresis, or capillary electrophoresis, or a combination thereof, and may further include washing for removing impurities, and concentration of the acquired vesicles.

The vesicles of the present invention may be isolated from the *L. plantarum* culture or the food manufactured by adding *L. plantarum,* or may be naturally secreted or artificially produced from *L. plantarum,* but the present invention is not limited thereto.

In the present invention, the vesicles isolated by the above method may have an average diameter of 10 to 1000 nm, 10 to 900 nm, 10 to 800 nm, 10 to 700 nm, 10 to 600 nm, 10 to 500 nm, 10 to 400 nm, 10 to 300 nm, 10 to 220 nm, 10 to 200 nm, 10 to 100 nm, 10 to 90 nm, 10 to 80 nm, 10 to 70 nm, 10 to 60 nm, 10 to 50 nm, 10 to 40 nm, or 20 to 40 nm, but the present invention is not limited thereto.

The term "included as an active ingredient" used herein refers to including a material in a sufficient amount to achieve the efficacy or activity of the *L. plantarum-*derived vesicles.

The term "immune disease or metabolic disease" used herein encompasses diseases occurring due to immune and metabolic dysfunction in the body. Generally, in immune diseases, inflammation is caused by pathogenic factors, and as a result, cell death and inflammatory cell infiltration mediated by an inflammatory mediator are induced. In addition, in metabolic diseases, inflammation occurs or metabolic stress is triggered due to a pathogenic metabolite produced in the body, resulting in cellular aging or cell death.

The immune or metabolic disease may be a disease caused by immune or metabolic dysfunction, but the present invention is not limited thereto.

The immune or metabolic disease includes one or more gastrointestinal diseases selected from the group consisting of gastritis, peptic ulcers, celiac disease, and inflammatory bowel disease;
one or more oral diseases selected from the group consisting of gingivitis, periodontitis, and caries;
one or more female vaginal diseases selected from the group consisting of candida vaginitis, atrophic vaginitis, bacterial vaginitis or vaginosis, and trichomonas vaginitis;
one or more liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more lung diseases selected from the group consisting of chronic obstructive pulmonary disease, chronic interstitial pneumonitis, emphysema, bronchopulmonary dysplasia, and idiopathic pulmonary fibrosis;
one or more cardiovascular diseases selected from the group consisting of hyperinsulinemia, dyslipidemia, arrhythmia, atherosclerosis, angina, metabolic syndrome, myocardial infarction, stroke, and cardiomyopathy;
one or more renal diseases selected from the group consisting of glomerulonephritis, and chronic nephropathy;
one or more musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis; and
one or more neurological diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

In addition, the present invention is provides a food composition for preventing or alleviating an immune disease or metabolic disease, comprising *Lactobacillus plantarum-derived* vesicles as an active ingredient.

The food composition may be a health functional food composition, but is not limited thereto.

The vesicles according to the present invention may be used by adding the vesicles as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

In addition, the present invention relates to a pharmaceutical composition for preventing or treating aging or an aging-related disease, which comprises *Lactobacillus plantarum*-derived vesicles as an active ingredient.

In addition the present is a food composition for preventing or alleviating aging, comprising *Lactobacillus plantarum*-derived vesicles as an active ingredient.

In addition the present is a cosmetic composition for preventing or alleviating skin aging, comprising *Lactobacillus plantarum*-derived vesicles as an active ingredient.

In the present invention, aging is a generic term for all physiological changes in the body that occur over time, and means a life phenomenon that occurs in various ways depending on an individual due to various factors. Specifically, the aging phenomenon means changes in functions of organs and tissue, and the aging of an individual is ultimately caused by the aging of cells constituting the individual. In the present invention, the aging may be aging of the brain, the liver, the lungs, the kidneys, muscles, skin, or cells of these organs, but the present invention is not limited thereto.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

The water-soluble vitamin may be any substance that is blendable with cosmetics, but examples thereof include vitamin B1, vitamin B2, vitamin B6, pyridoxine, pyridoxine hydrochloride, vitamin B 12, pantothenic acid, nicotinic acid, nicotinic acid amide, folic acid, vitamin C, vitamin H, and the like, and salts thereof (thiamine hydrochloride, sodium ascorbate, and the like) or derivatives thereof (sodium ascorbic acid-2-phosphate, magnesium ascorbic acid-2-phosphate, and the like) are also included in water-soluble vitamins that may be used in the present invention. These water-soluble vitamins may be obtained by a conventional method such as microbial transformation, purification from a microbial culture, an enzyme method, or a chemical synthesis method.

The oil-soluble vitamins may be any substance that is blendable with cosmetics, but examples thereof include vitamin A, carotene, vitamin D2, vitamin D3, vitamin E (d1-α-tocopherol, d-α-tocopherol), or the like, and derivatives thereof (e.g., ascorbyl palmitate, ascorbyl stearate, ascorbyl dipalmitate, d1-α-tocopherol acetate, d1-α-tocopherol nicotinate, vitamin E, DL-pantothenyl alcohol, D-pantothenyl alcohol, pantothenyl ethylether) may also be included in the oil-soluble vitamins used in the present invention. These oil-soluble vitamins may be obtained by a conventional method such as microbial transformation, purification from a microbial culture, or enzymatic or chemical synthesis.

The polymer peptides may be any substance that is blendable with cosmetics, but examples thereof may include collagen, hydrolyzed collagen, gelatin, elastin, hydrolyzed elastin, and keratin. The polymer peptides may be purified and obtained by any conventional method such as purification from a microbial culture, an enzyme method, or a chemical synthesis method, or may generally be used by being purified from natural substances such as the dermis of a pig, a cow, or the like and silk fiber of silkworms.

The polymeric polysaccharides may be any substance that is blendable with cosmetics, and examples thereof may include hydroxyethyl cellulose, xanthan gum, sodium hyaluronate, and chondroitin sulfate or salts thereof (sodium salts). For example, chondroitin sulfate or salts thereof may generally be purified from mammals or fish and used.

The sphingolipids may be any substance that is blendable with cosmetics, and examples thereof may include ceramide, phytosphingosine, and sphingoglycolipid. The sphingolipids may be purified, by a conventional method, from mammals, fish, shellfish, yeast, or plants, or may be obtained by a chemical synthesis method.

The cosmetic composition of the present invention may include, as necessary, other ingredients mixed in conventional cosmetics along with the above essential ingredients.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alpha-olefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/methylcetyloxysiloxane copolymers, dimethylsiloxane/methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyltaurine.; N-acyl alkaline amino acids such as N-epsilon-lauroyl-L-lysine, N-epsilonpalmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

In addition, the present invention is a composition for delivering a drug for treating an immune disease or metabolic disease, comprising *Lactobacillus plantarum-*derived vesicles as an active ingredient.

As used herein, the term "drug delivery" refers to all means or actions which load and deliver a drug to the vesicle according to the present invention in order to deliver the drug to a specific organ, tissue, cell, or organelle.

In the present invention, the composition for delivering a drug may deliver the drug to one or more organs selected from the group consisting of the stomach, small intestine, large intestine, lungs, liver, kidneys, and brain, but is not limited thereto.

Further, the present invention provides a method for delivering a drug for treating an immune disease or metabolic disease, the method comprising administering a composition comprising *Lactobacillus plantarum*-derived vesicles, which carry a drug for an immune disease or metabolic disease, as an active ingredient to a subject in need thereof.

In addition, the present invention provides a use of a composition comprising *Lactobacillus plantarum*-derived vesicles as an active ingredient for delivering a therapeutic drug for an immune disease or metabolic disease.

In addition, the present invention provides a use of vesicles derived from *Lactobacillus plantarum* for preparing a preparation for delivering a drug for treating an immune disease or metabolic disease.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1. Isolation of Lactobacillus plantarum-derived vesicles

To isolate *Lactobacillus plantarum*-derived vesicles (extracellular vesicles; EV), the *Lactobacillus plantarum* strain was inoculated in a deMan-Rogosa and Sharpe (MRS) medium and cultured at 37 °C and 200 rpm until an absorbance (OD₆₀₀ₙₘ) became 1.0 to 1.5, and then reinoculated in a Luria Bertani (LB) medium and cultured. Afterward, the culture medium containing the bacterial cells was recovered and centrifuged at 10,000 g and 4 °C for 20 minutes, thereby obtaining a supernatant from which the bacterial cells were removed. The obtained supernatant was then filtrated through a 0.22 µm filter, and the filtered supernatant was concentrated to a volume of 50 mL or less using a 100 kDa Pellicon 2 Cassette filter membrane (Merck Millipore) and a MasterFlex pump system (Cole-Parmer). The concentrated supernatant was filtrated again through a 0.22 µm filter to isolate *Lactobacillus plantarum*-derived vesicles. In the following examples, experiments were conducted using the isolated vesicles.

### Example 2. Confirmation of pharmacokinetic properties of Lactobacillus plantarum-derived vesicles

To confirm the pharmacokinetic properties such as absorption and distribution patterns after orally administering *Lactobacillus plantarum*-derived vesicles, distribution in the body was confirmed in the following manner.

*Lactobacillus plantarum*-derived vesicles labeled with the fluorophore Cy7 were orally administered to a mouse, and after 0, 1, 3, 6, 24, or 48 hours, the pattern of infiltration into various organs was evaluated. Whole body imaging was performed on the mouse using optical imaging equipment (Davinch-In vivo Fluoro Chemi (western); Davinch-K) to observe a fluorescence signal. In addition, to evaluate the distribution pattern to major organs after vesicle administration, the brain, blood, the heart, the lungs, the liver, the stomach, the spleen, the small intestine, the large intestine, and the kidneys were collected, and the collected tissue was observed using optical imaging equipment (Davinch-In vivo Fluoro Chemi (western); Davinch-K) to observe a fluorescence signal.

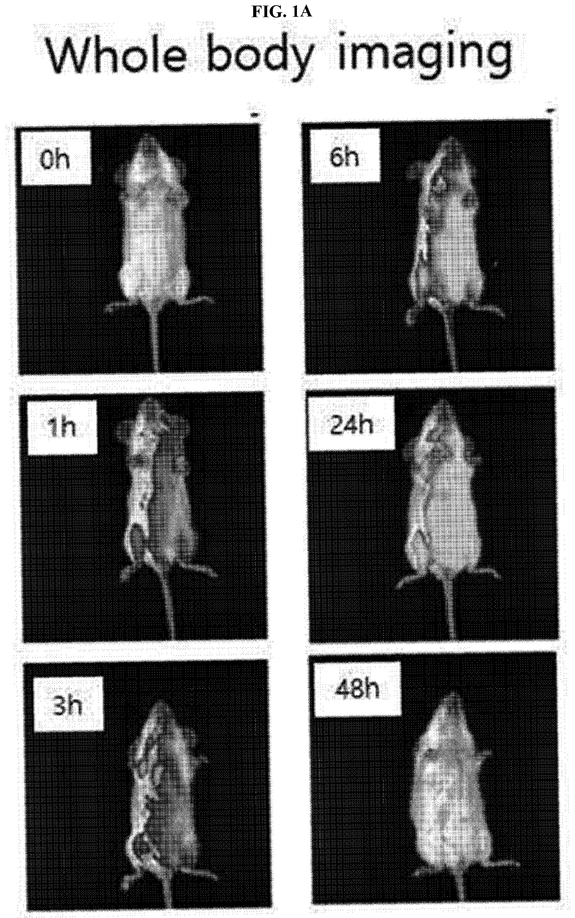

As shown in FIG. 1A, it was confirmed that the vesicles were absorbed systemically one hour after vesicle administration, which continued until 24 hours after administration, and most of the vesicles were excreted after 48 hours.

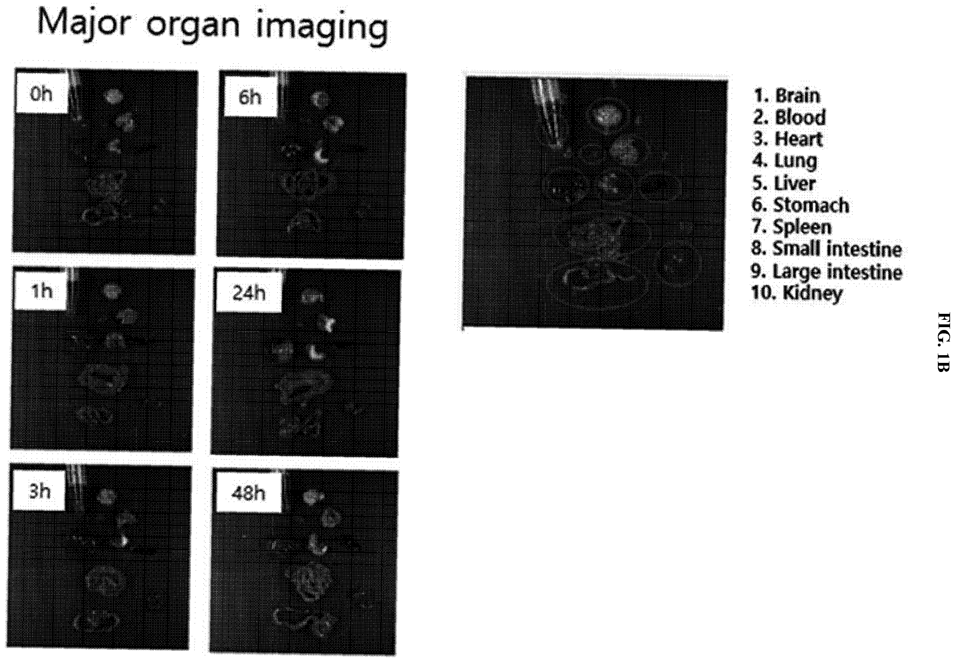

In addition, as shown in FIG. 1B, it was confirmed that the vesicles were absorbed into the stomach only one hour after vesicle administration, distributed in the stomach, the small intestine, the large intestine, and the lungs after 3 hours, and 6 hours after administration, distributed in the liver, the kidneys and the brain. Meanwhile, it was confirmed that the vesicles were continuously distributed in various organs more than 24 hours after administration, and then after 48 hours, most of the vesicles were secreted.

### Example 3. Confirmation of inhibitory effect of Lactobacillus plantarum-derived vesicles on cell death caused by pathogenic factor

A biological factor causing a disease is recognized by a pattern recognition receptor (prr) of cells to form an inflammasome, and thus induces not only cell death (pyroptosis) but also inflammatory cell infiltration by an inflammatory mediator to accelerate cell death, resulting in organ failure. In the present invention, the therapeutic effect of *Lactobacillus plantarum-derived* vesicles on cell death caused by a biological factor was evaluated.

To this end, the killing effects on epithelial cells were compared by treating skin epithelial cells (HaCaT cells) with *Staphylococcus aureus*-derived vesicles (Sa EV), which are pathogenic factors, and *Lactobacillusplantarum*-derived vesicles (Lp EV) at different concentrations.

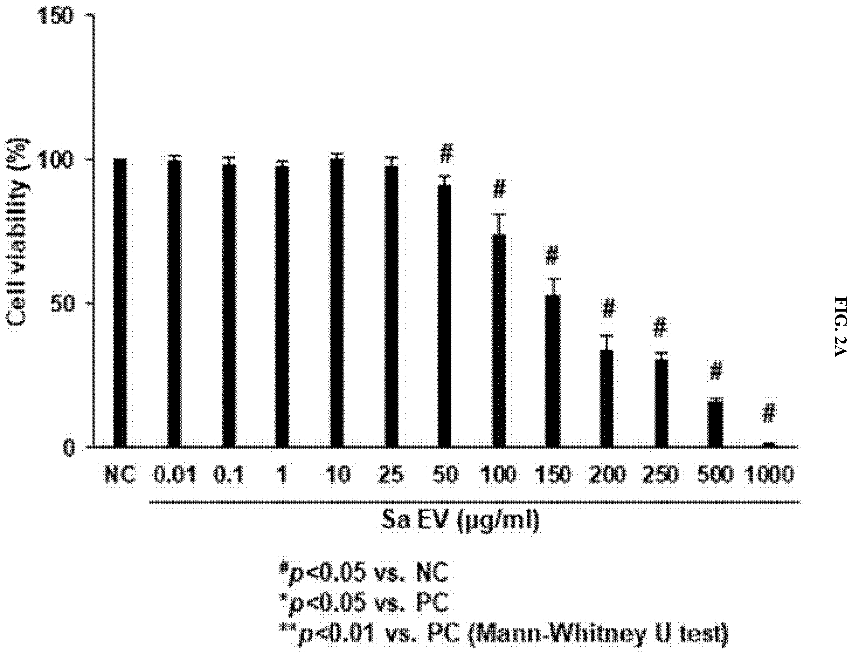

As a result, as shown in FIG. 2A, cell death was induced by the *Staphylococcus aureus*-derived vesicles in a dose-dependent manner.

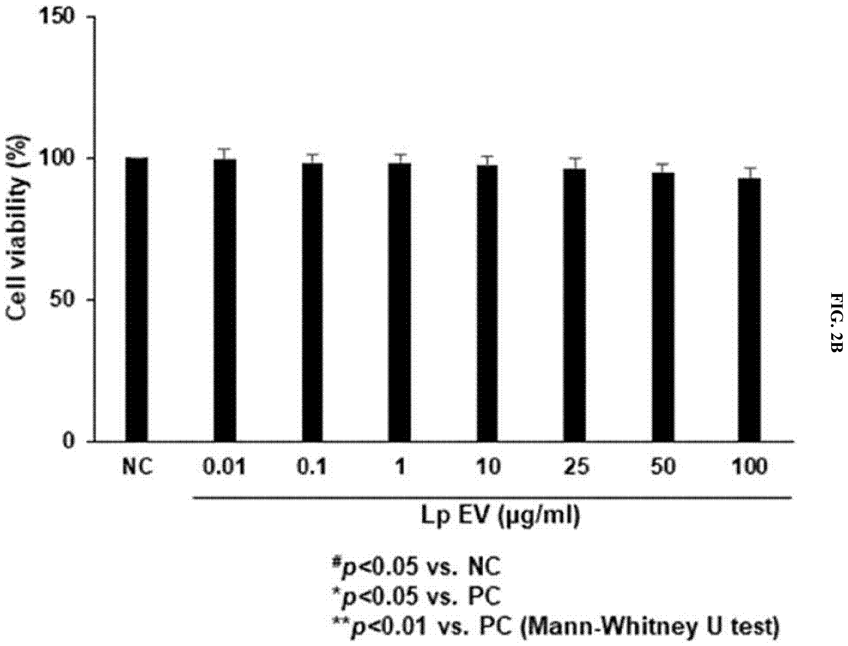

On the other hand, as shown in FIG. 2B, the cell death of epithelial cells was not induced by *Lactobacillus plantarum*-derived vesicles.

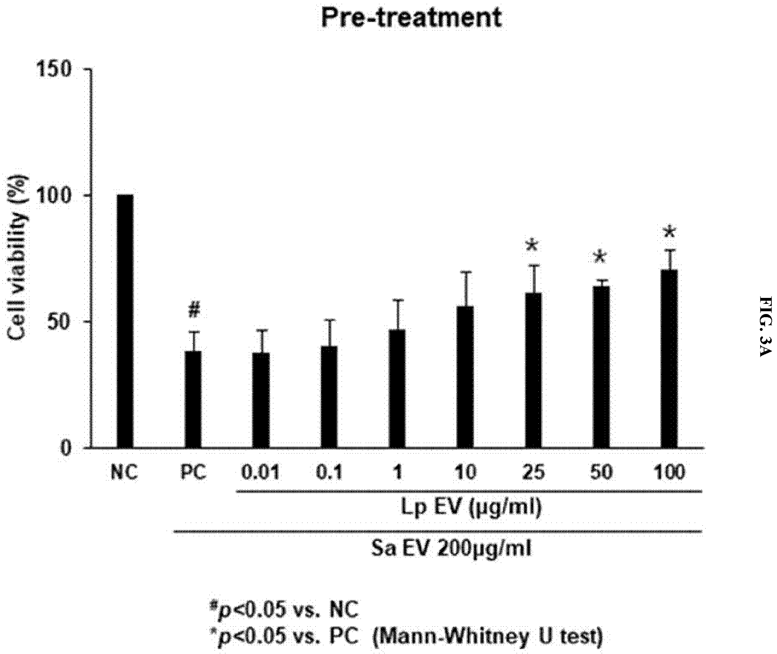

As shown in FIG. 3A, when epithelial cells were treated with *Lactobacillus plantarum*-derived vesicles before treatment with *Staphylococcus aureus*-derived vesicles, the death of the epithelial cells was inhibited by the *Lactobacillus plantarum-*derived vesicles in a dose-dependent manner.

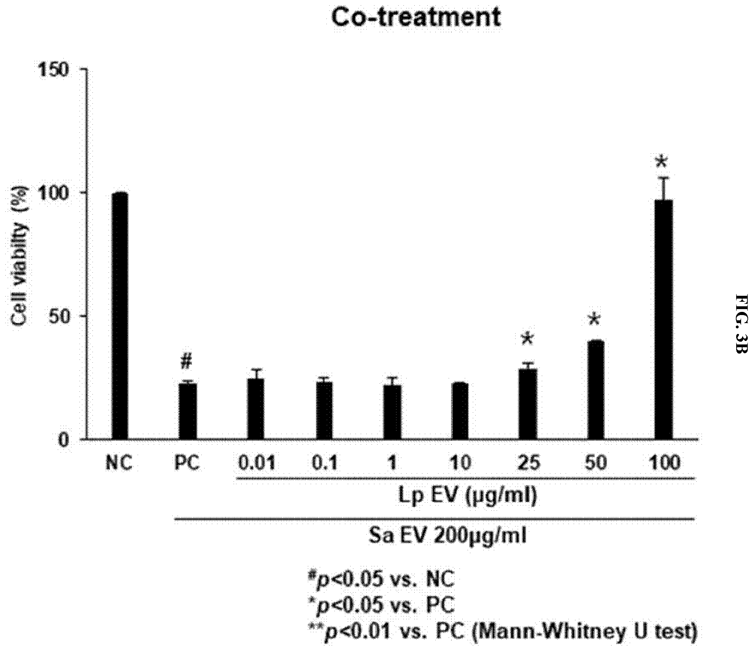

In addition, as shown in FIG. 3B, when epithelial cells were co-treated with *Staphylococcus aureus*-derived vesicles and *Lactobacillus plantarum*-derived vesicles, the death of epithelial cells was inhibited by the *Lactobacillus plantarum*-derived vesicles in a dose-dependent manner.

The above results showed that cell death caused by biological pathogenic factors is significantly inhibited by *Lactobacillus plantarum*-derived vesicles.

### Example 4. Confirmation of therapeutic effect of Lactobacillus plantarum-derived vesicles in disease mouse induced by dextran diet

Recently, with changes in dietary patterns, many people have diets containing large amounts of sugar (sucrose). When sucrose is ingested, certain bacteria with dextransucrase in the oral cavity produce dextran using sucrose as a raw material. Dextran produced by consuming food containing a large amount of sucrose is not digested by enzymes in the intestines and moves to the large intestine, causing colitis.

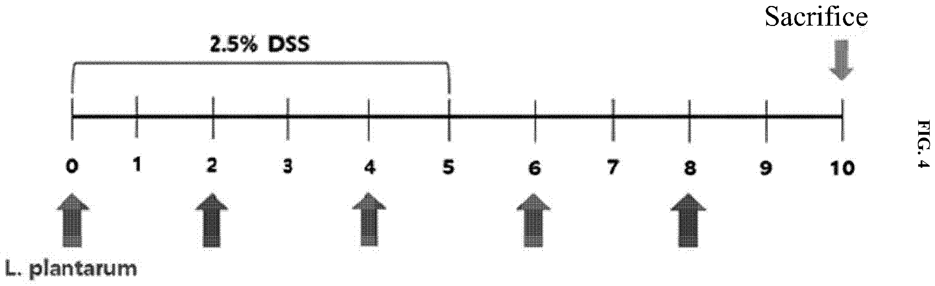

Accordingly, to evaluate the therapeutic effect of *Lactobacillus plantarum-*derived vesicles on metabolic dysfunction induced by sucrose, 2.5% dextran sulfate sodium (DSS) was orally administered to a mouse (8-week-old C57BL/6N male mouse) for 5 days to create a colitis model (see FIG. 4). In addition, after co-administering DSS and *Lactobacillusplantarum*-derived vesicles (50 µg/mouse) daily for 5 days and then administering the *Lactobacillusplantarum*-derived vesicles every two days, an anatomical change occurring in the large intestine on day 10 after dextran administration was evaluated.

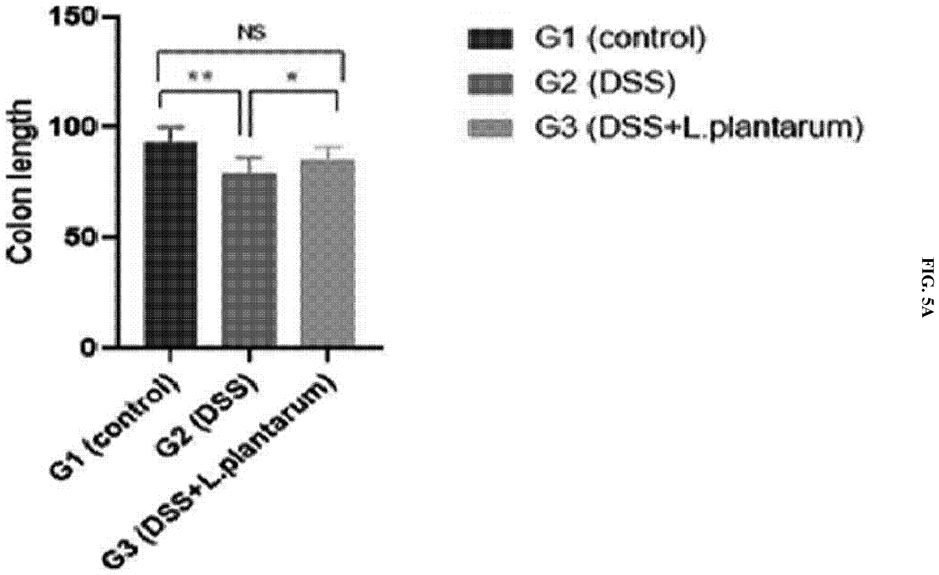

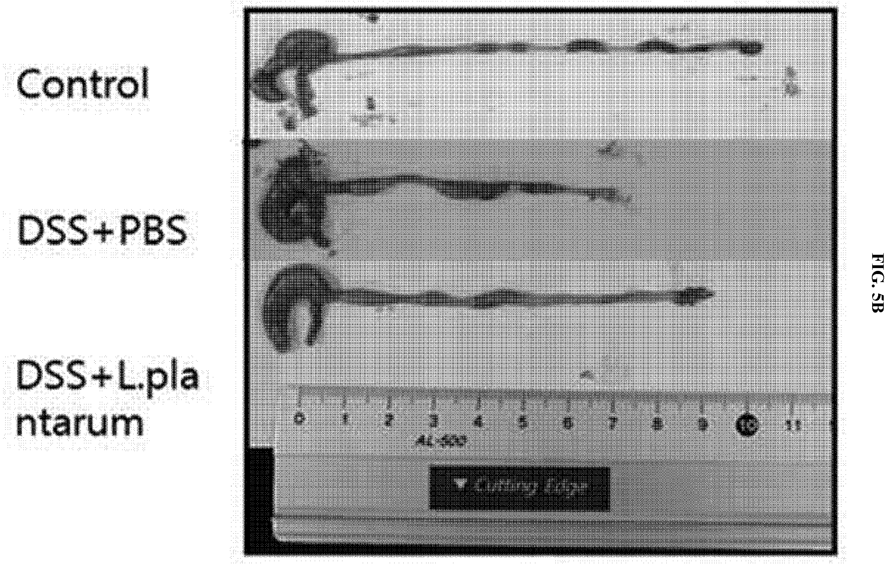

As a result, as shown in FIGS. 5A and 5B, the length of the large intestine of the colitis mouse model was significantly reduced compared to that of a normal mouse (control). In addition, when *Lactobacillus plantarum*-derived vesicles were administered to a DSS-administered mouse, the length of the large intestine was restored to the normal mouse level.

The above results showed that an anatomical change in colitis caused by dextran, which is an abnormal metabolite of sucrose, is significantly inhibited and improved by *Lactobacillus plantarum*-derived vesicles.

### Example 5. Confirmation of therapeutic effect of Lactobacillus plantarum-derived vesicles on expression of neurogenesis-related neurotrophin gene

When neural stem cells and nerve cells are repeatedly exposed to various mental stresses, it is known that neurogenesis ability decreases. Neurotrophins are a group of growth factor proteins that play a critical role not only in the survival and function of differentiated nerve cells but also in the development of neural stem cells. The brains of mammals, including humans, usually produce nerve cells during the fetal period, but due to the presence of neural stem cells in the hippocampus and striatum areas, adult neurogenesis occurs even in adulthood. Representative examples of neurotrophins associated with the survival and function of neural stem cells and nerve cells are brain-derived neurotrophic factor (BDNF), neurotrophin (NT)-3, NT4/5, and a nerve growth factor (NGF). Among these, BDNF is a protein that is present in the central nervous system and the peripheral nervous system, and not only increases the survival and synapse formation of nerve cells in the brain and the peripheral nervous system through the TrkB receptor, but also induces the proliferation and differentiation of neural stem cells.

Therefore, to evaluate the therapeutic effect of *Lactobacillus plantarum-*derived vesicles on the survival and function of neural stem cells and nerve cells caused by mental stress, hippocampal nerve cells (HT22 cells) were cultured in a Dulbecco's modified Eagles (DMEM) medium, and an adrenocortical hormone (glucocorticoid (GC), 400 ng/mL) was administered to suppress neurotrophin gene expression. In addition, the nerve cells were also treated with *Lactobacillus plantarum*-derived vesicles (20 µg/mL) to evaluate a therapeutic effect. As a method of evaluating gene expression, the cells were lysed using a lysis buffer to extract genes, and gene expression was quantified using RT-PCR. The degree of gene expression was evaluated using specific primers for total Bdnf (tBdnf), Bdnfl, Bdnf4, and Ngf mRNAs. The specific sequences of the primers are shown in Table 1 below. A group not treated with GC is denoted as CON, and a control (vehicle) indicates a group not including vesicles.

**[Table 1]**

| mRNA | Sequence | SEQ. ID. NO |
|---|---|---|
| total Bdnf | F: TGGCTGACACTTTTGAGCAC | 1 |
| | R: GTTTGCGGCATCCAGGTAAT | 2 |
| Bdnf1 | F: CCTGCATCTGTTGGGGAGAC | 3 |
| | R: GCCTTGTCCGTGGACGTTTA | 4 |
| Bdnf4 | F: CAGAGCAGCTGCCTTGATGTT | 5 |
| | R: GCCTTGTCCGTGGACGTTTA | 6 |
| Ngf | F: AGCATTCCCTTGACACAG | 7 |
| | R:GGTCTACAGTGATGTTGC | 8 |
| Sirt1 | F: GATCCTTCAGTGTCATGGTTC | 9 |
| | R: ATGGCAAGTGGCTCATCA | 10 |
| Hdac2 | F: GGGACAGGCTTGGTTGTTTC | 11 |
| | R: GAGCATCAGCAATGGCAAGT | 12 |
| Creb1 | F: GAGGCAGCAAGAGAATGTCG | 13 |
| | R: CCAGTCCATTCTCCACCGTA | 14 |

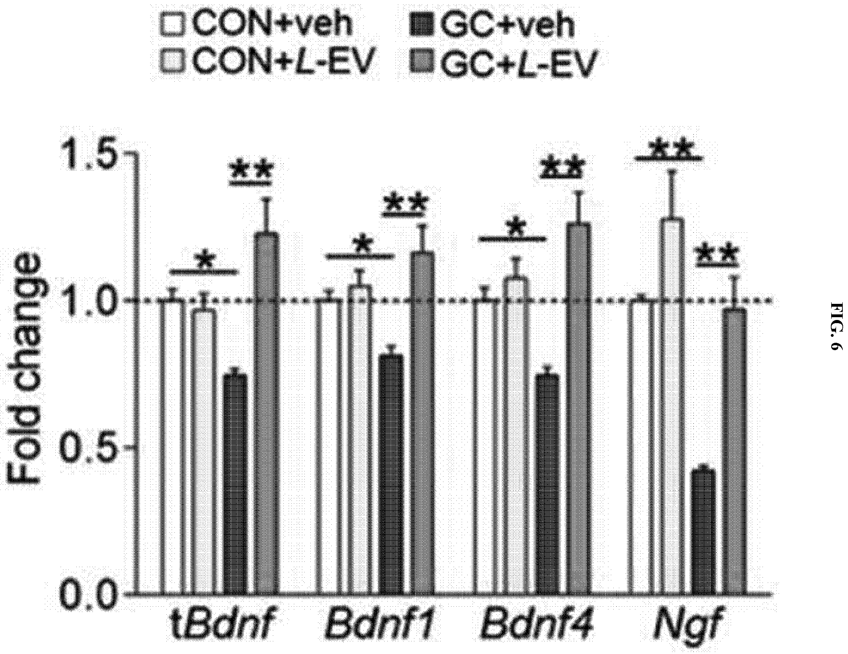

As a result, as shown in FIG. 6, when nerve cells were treated with GC, the expression of tBdnf, Bdnfl, Bdnf4, and Ngf genes was significantly suppressed. On the other hand, the expression of tBdnf, Bdnfl, Bdnf4, and Ngf genes, suppressed by GC, was improved to a normal level or higher when administering *Lactobacillus plantarum*-derived vesicles.

The above results showed that *Lactobacillus plantarum*-derived vesicles can increase the expression of neurotrophin genes to induce neurogenesis.

### Example 6. Confirmation of therapeutic effect of Lactobacillus plantarum-derived vesicles on expression of aging-related gene

When repeatedly exposed to various stresses, genetic damage occurs due to oxidative stress within cells, resulting in cellular aging and abnormal cell death. Sirtuin is a key signaling protein that maintains cellular homeostasis following cell damage in stress situations. Among sirtuin proteins, sirtuin 1 (Sirt1) is present in the nucleus and cytoplasm, and as a histone deacetylase, it is known as an anti-aging protein that increases cell survival by treating gene damage caused by stress.

Therefore, to evaluate the therapeutic effect of *Lactobacillus plantarum-*derived vesicles on cellular aging, after treating hippocampal nerve cells (HT22 cells) with *Lactobacillus plantarum*-derived vesicles by the method described in Example 5, it was evaluated whether the expression of the sirtuin gene suppressed by GC could be restored. The level of gene expression was assessed using specific primers for Sirt1, histone deacetylase 2 (Hdac2), or CAMP responsive element binding protein 1 (Creb 1).

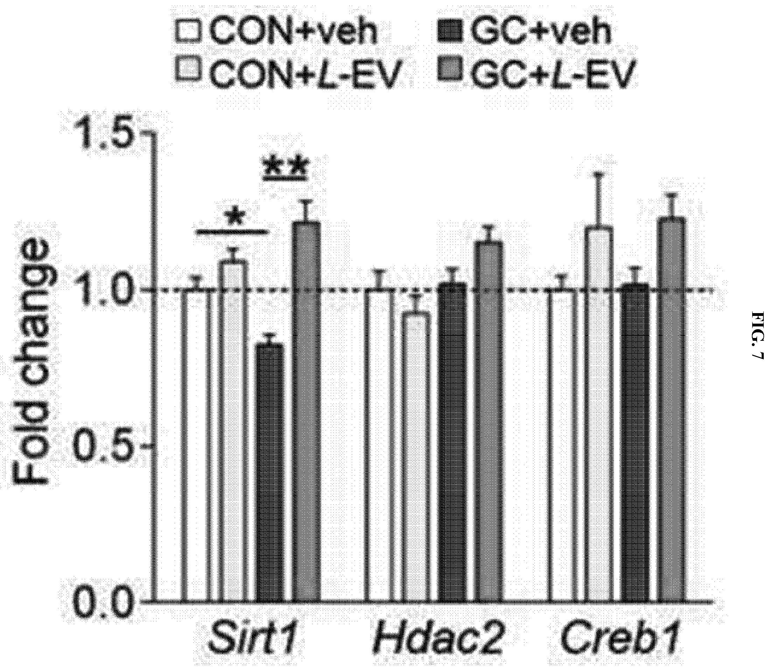

As a result, as shown in FIG. 7, Sirt1 gene expression in nerve cells was significantly suppressed by GC, and when *Lactobacillus plantarum*-derived vesicles were administered, the expression was restored to a normal level. The above results can show that *Lactobacillusplantarum*-derived vesicles enhance Sirt1 gene expression to inhibit cellular aging and cell death caused by genetic damage due to various stresses.

### Example 7. Confirmation of effect of Lactobacillus plantarum-derived vesicles in maintaining cellular homeostasis against metabolic stress

Cellular aging is defined as the loss of cell division ability due to repetitive physical, chemical, biological, or mental stress. As cellular aging caused by repetitive stress and cell regeneration ability decrease, aging-related diseases occur. Particularly, cells induce the metabolic homeostasis of cells by activating the AMP-activated protein kinase (AMPK) protein as a signaling pathway in cells in a metabolic stress situation where ATP production decreases, thereby inhibiting cellular aging and death. That is, AMPK signaling inhibits metabolic dysfunction that occurs during energy depletion through a mechanism such as autophagy.

Therefore, an experiment was conducted to evaluate whether *Lactobacillus plantarum*-derived vesicles (Lp EV) affect cellular aging through AMPK activation in cells. To evaluate AMPK activation according to a concentration of treated *Lactobacillus plantarum*-derived vesicles *in vitro,* muscle cells (L6 myotube cells) were treated with the *Lactobacillus plantarum*-derived vesicles at 0, 0.1, 1, or 10 µg/mL for 1 hour. As a control, insulin (1 µM), which promotes aging and metformin (50 mM), which inhibits aging were used. After treating the cells with the drug, the difference in the amount of pAMPK, which is a key indicator in AMPK signaling, was measured through western blotting. First, serum-free DMEM in which 2 × 10⁶ of the cells were seeded was added to a 60 mm cell culture petri dish and cultured for 2 hours. Afterward, 0, 0.1, 1, or 10 µg/mL of *Lactobacillus plantarum*-derived vesicles were treated for 1 hour, and insulin (1 µM) and metformin (50 mM) as controls were also treated for 1 hour. To perform a cell lysis assay, the sample-treated petri dish was put on ice, a supernatant was suctioned, and cold PBS buffer was added thereto at 5 mL and washed twice. 10 µL of a protease/phosphatase inhibitor was added to 1 mL of lysis buffer and well mixed, and 100 µL of cell lysis buffer was dropped on each dish, followed by incubation on ice for 5 minutes. After detaching the cells with a scraper, the resulting cells were transferred to a 1.5 mL microtube, followed by repeating vortexing and incubation on ice 1 minute each for 20 minutes. Subsequently, centrifugation was performed at 14,000 rpm for 10 minutes at 4 °C, and a supernatant of the lysed sample was transferred to two new 1.5 mL microtubes at 5 µL and 70 µL, respectively. The microtube containing 5 µL of the supernatant was stored at -20 °C, and 16.5 µL of 5X sample buffer was added to the microtube containing 70 µL of the supernatant and then boiled at 100 °C for 5 minutes. The boiled sample was stored at -20 °C. To perform BCA quantitative analysis, the 1.5 mL microtube containing 5 µL of the lysed sample supernatant was taken out at room temperature, 20 µL of sterile distilled water was added thereto, and the resulting solution was vortexed and then spun down. Bovine serum albumin (BSA) was dissolved in sterile distilled water at 2 mg/mL and then diluted by 1/2 to prepare 2, 1, 0.5, 0.25, 0.125, 0.0625, 0.03125, and 0 mg/mL stocks. 25 µL of BSA was added to each of three wells of a 96-well polystyrene plate at different concentrations, and 25 µL of the lysed sample was also added to each well. 8 mL of BCA Protein Assay Reagent A and 160 µL of BCA Protein Assay Reagent B were mixed, and then added to each well at 200 µL. After reacting in a 37 °C incubator for 30 minutes, absorbance was measured at 562 nm using a SpectraMax M3 microplate reader (Molecular Devices, USA). For western blotting, in the process of tris-glycine SDS-polyacrylamide gel electrophoresis, a 10% gel was prepared, and 50 µg of a protein for each sample was quantified and loaded. After transfer to a nitrocellulose membrane and going through a blocking process with 5% skim milk, AMPKα antibodies were diluted 1:400, phospho-AMPKα antibodies were diluted 1:400, and β-actin antibodies were diluted 1: 1,000 in 5% skim milk, and then mixed with the membrane overnight. After washing with a 1X PBST (0.05% Tween-20-containing PBS) solution three times for 5 minutes each, anti-rabbit IgG and HRP-linked antibodies were diluted 1:1,000 and mixed with the membrane for 1 hour. After washing with a 1X PBST solution three times for 5 minutes each, a solution in which solution A and solution B of a West-Q Chemiluminescent Substrate Kit were mixed 1:1 was sufficiently sprayed on the membrane and bands were confirmed with a Chemidoc device.

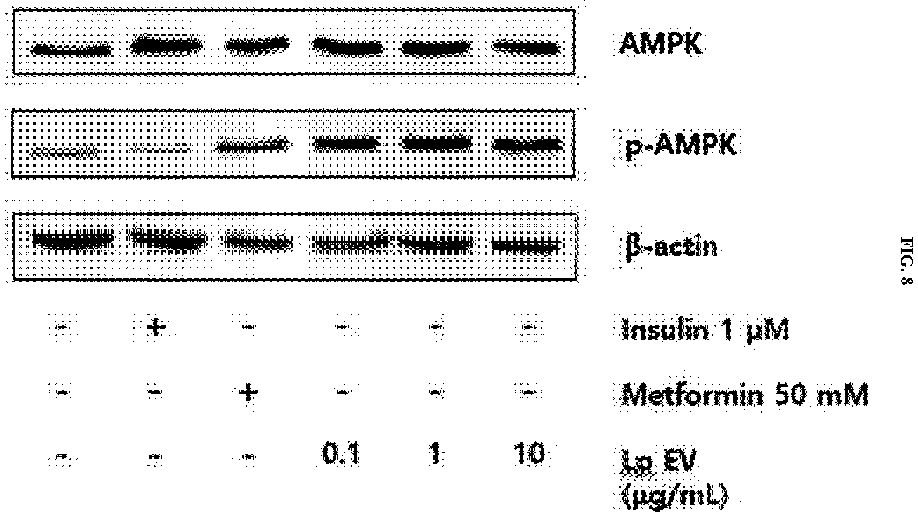

As a result, as shown in FIG. 8, phosphorylation of AMPK (pAMPK) was inhibited by insulin as a control drug, and increased by metformin. In addition, when *Lactobacillus plantarum*-derived vesicles were treated, pAMPK expression was increased in an administration dose-dependent manner.

The above results can show that *Lactobacillus plantarum*-derived vesicles increase cellular homeostasis against metabolic stress through the activation of AMPK signaling to enhance cell viability.

### Example 8. Confirmation of effect of Lactobacillus plantarum-derived vesicles on secretion of inflammatory mediator by pathogenic factor

To confirm the inflammation-regulating effect of *Lactobacillus plantarum-*derived vesicles, a mouse macrophage cell line Raw 264.7 was pretreated with *Lactobacillus plantarum*-derived vesicles at each concentration of 0.01, 0.1, and 1 µg/mL and cultured, the secretion amounts of IL-6 and TNF-α, which are inflammatory mediators, were measured in macrophages stimulated by pathogenic vesicles, that is, *E. coli*-derived vesicles (*E*. *coli* EV). More specifically, after seeding Raw 264.7 cells in a 48-well plate, the cells were treated with *Lactobacillus plantarum*-derived vesicles diluted with serum-free DMEM and cultured for 12 hours, and then the cells were treated with 1 µg/mL of E. coli-derived vesicles and cultured for an additional 12 hours. The secretion amounts of IL-6 and TNF-α were measured in the cell culture obtained by the above method by ELISA.

For ELISA, capture antibodies were diluted in PBS, dispensed at 50 µL in each well of a 96-well polystyrene plate according to the working concentration, and reacted at 4 °C overnight. Afterward, each well was washed with 100 µL of a PBST (0.05 % Tween-20-containing PBS) solution twice, 100 µL of an RD (1 % BSA-containing PBS) solution was dispensed to perform blocking at room temperature for 1 hour, washing was performed twice with 100 µL of PBST, and 50 µL each of the sample and the standard was dispensed according to the concentration at room temperature for 2 hours. After washing again with 100 µL of PBST twice, detection antibodies were diluted in RD and dispensed according to the working concentration at 50 µL and allowed to react room temperature for 2 hours. In addition, after washing again with 100 µL of PBST twice, streptavidin-HRP was diluted in RD 1/200 and dispensed at 50 µL and allowed to react at room temperature for 30 minutes. Finally, after washing 100 µL of PBST three times, 50 µL of a 1:1 mixture of a TMB substrate and 0.04% hydrogen peroxide was dispensed, and then a sufficient amount of time was allowed to pass for color development. When color development progressed for 5 to 20 minutes, 50 µL of a 1M sulfuric acid solution was dispensed to stop the reaction, and absorbance was measured at 450 nm using a Synergy^{™} HT multi-detection microplate reader (BioTek, USA).

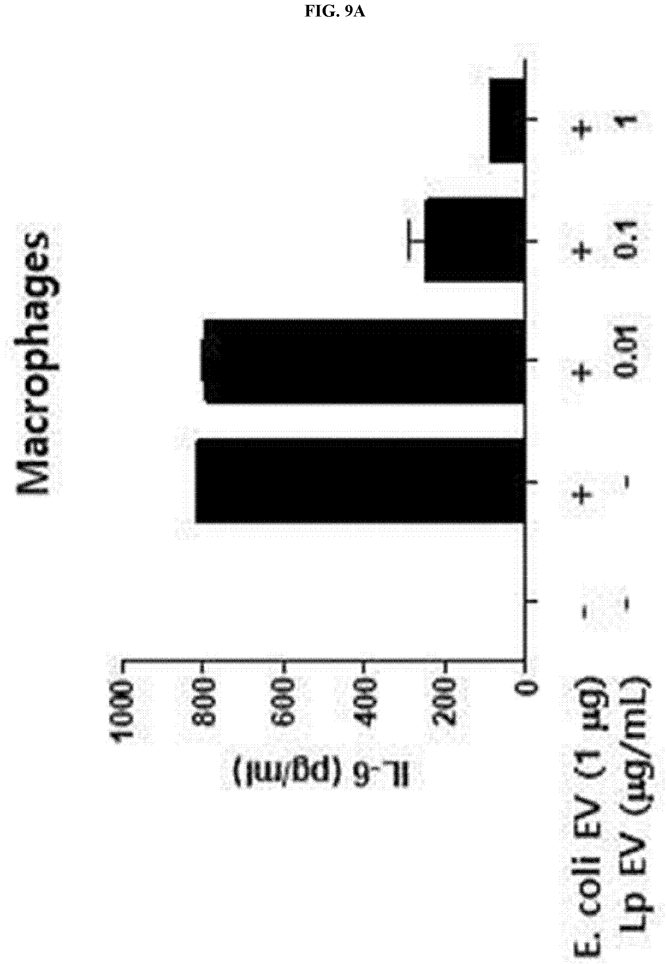

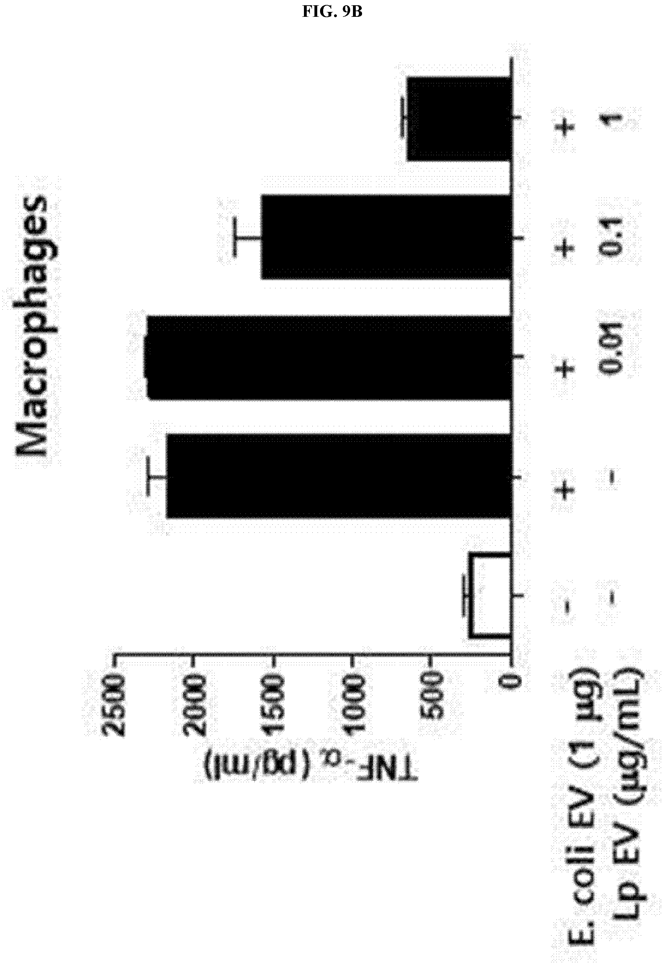

As a result, as shown in FIGS. 9A and 9B, it was confirmed that, in a *Lactobacillus plantarum*-derived vesicle-treated sample, the secretion of IL-6 and TNF-α by *E*. *coli*-derived vesicles was decreased in a dose-dependent manner. The above result confirmed that an inflammatory response induced by pathogenic bacteria-derived vesicles can be effectively inhibited using *Lactobacillus plantarum*-derived vesicles.

The above-described description of the present invention is merely provided to exemplify the present invention, and it will be understood by those of ordinary skill in the art to which the present invention belongs that the present invention can be implemented in modified forms without departing from the essential features of the present invention. Therefore, the exemplary embodiments described above should be interpreted as illustrative and not limited in any aspect.

### [Industrial Applicability]

*Lactobacillus plantarum*-derived vesicles according to the present invention can be effectively used not only for developing medicines or health functional food for preventing or treating an immune or metabolic disease or alleviating symptoms thereof but also effectively used as a drug delivery system for treating the disease.

## Claims

1. A pharmaceutical composition for preventing or treating an immune disease or metabolic disease, comprising *Lactobacillus plantarum-derived* vesicles as an active ingredient.

2. The pharmaceutical composition of claim 1, wherein the immune disease or metabolic disease is selected from one or more gastrointestinal diseases selected from the group consisting of gastritis, peptic ulcers, celiac disease, and inflammatory bowel disease;
one or more oral diseases selected from the group consisting of gingivitis, periodontitis, and caries;
one or more female vaginal diseases selected from the group consisting of candida vaginitis, atrophic vaginitis, bacterial vaginitis or vaginosis, and trichomonas vaginitis;
one or more liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more lung diseases selected from the group consisting of chronic obstructive pulmonary disease, chronic interstitial pneumonitis, emphysema, bronchopulmonary dysplasia, and idiopathic pulmonary fibrosis;
one or more cardiovascular diseases selected from the group consisting of hyperinsulinemia, dyslipidemia, arrhythmia, atherosclerosis, angina, metabolic syndrome, myocardial infarction, stroke, and cardiomyopathy;
one or more renal diseases selected from the group consisting of glomerulonephritis, and chronic nephropathy;
one or more musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis; and
one or more neurological diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy.

3. The pharmaceutical composition of claim 1, wherein the vesicles have an average diameter of 10 to 1000 nm.

4. The pharmaceutical composition of claim 1, wherein the vesicles are isolated from a culture medium of *Lactobacillus plantarum.*

5. The pharmaceutical composition of claim 1, wherein the vesicles are isolated from food manufactured by adding *Lactobacillus plantarum.*

6. The pharmaceutical composition of claim 1, wherein the vesicles are naturally secreted or artificially produced from *Lactobacillus plantarum.*

7. A food composition for preventing or alleviating an immune disease or metabolic disease, comprising *Lactobacillus plantarum-derived* vesicles as an active ingredient.

8. The food composition of claim 7, wherein the immune disease or metabolic disease is selected from one or more gastrointestinal diseases selected from the group consisting of gastritis, peptic ulcers, celiac disease, and inflammatory bowel disease;
one or more oral diseases selected from the group consisting of gingivitis, periodontitis, and caries;
one or more female vaginal diseases selected from the group consisting of candida vaginitis, atrophic vaginitis, bacterial vaginitis or vaginosis, and trichomonas vaginitis;
one or more liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more lung diseases selected from the group consisting of chronic obstructive pulmonary disease, chronic interstitial pneumonitis, emphysema, bronchopulmonary dysplasia, and idiopathic pulmonary fibrosis;
one or more cardiovascular diseases selected from the group consisting of hyperinsulinemia, dyslipidemia, arrhythmia, atherosclerosis, angina, metabolic syndrome, myocardial infarction, stroke, and cardiomyopathy;
one or more renal diseases selected from the group consisting of glomerulonephritis, and chronic nephropathy;
one or more musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis; and
one or more neurological diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy.

9. The food composition of claim 7, wherein the vesicles are isolated from a culture medium of *Lactobacillus plantarum.*

10. The food composition of claim 7, wherein the vesicles are isolated from food manufactured by adding *Lactobacillus plantarum.*

11. The food composition of claim 7, wherein the vesicles are naturally secreted or artificially produced from *Lactobacillus plantarum.*

12. A pharmaceutical composition for preventing or treating aging, comprising *Lactobacillus plantarum-derived* vesicles as an active ingredient.

13. A food composition for preventing or alleviating aging, comprising *Lactobacillus plantarum-derived* vesicles as an active ingredient.

14. A cosmetic composition for preventing or alleviating skin aging, comprising *Lactobacillus plantarum-derived* vesicles as an active ingredient.

15. A composition for delivering a drug for treating an immune disease or metabolic disease, comprising *Lactobacillus plantarum-derived* vesicles as an active ingredient.

16. A method for preventing or treating an immune disease or metabolic disease, the method comprising administering a composition comprising vesicles derived from *Lactobacillus plantarum* as an active ingredient to a subject in need thereof.

17. A use of a composition comprising vesicles derived from *Lactobacillus plantarum* as an active ingredient for preventing or treating an immune disease or metabolic disease.

18. A use of vesicles derived from *Lactobacillus plantarum* for preparing a drug for treating an immune disease or metabolic disease.

19. A method for preventing or treating aging, the method comprising administering a composition comprising vesicles derived from *Lactobacillus plantarum* as an active ingredient to a subject in need thereof.

20. A use of a composition comprising vesicles derived from *Lactobacillus plantarum* as an active ingredient for preventing or treating aging.

21. A use of vesicles derived from *Lactobacillus plantarum* for preparing a drug for treating aging.

22. A method of delivering a drug for treating an immune disease or metabolic disease, which comprises administering a composition comprising *Lactobacillus plantarum-derived* vesicles carrying a desired immune disease or metabolic disease drug as an active ingredient to a subject in need thereof.

23. A use of a composition comprising *Lactobacillus plantarum-derived* vesicles as an active ingredient for delivering a drug for treating an immune disease or metabolic disease.

24. A use of *Lactobacillus plantarum-derived* vesicles for preparing a preparation for delivering a drug for treating an immune disease or metabolic disease.
